# EUROPÄISCHE PATENTSCHRIFT

(11) **EP 1 542 801 B1**
(45) Veröffentlichungstag und Bekanntmachung des Hinweises auf die Patenterteilung: **29.12.2010**
(21) Anmeldenummer: 03753324.7
(22) Anmeldetag: 23.09.2003
(51) Int. Cl.: B01L 3/00, G01N 31/22

(54) **VORRICHTUNG ZUR ERMITTLUNG UND ANZEIGE MINDESTENS EINER PHYSIKALISCHEN, CHEMISCHEN ODER BIOLOGISCHEN EIGENSCHAFT EINER TESTFLÜSSIGKEIT**
DEVICE FOR DETERMINING AND DISPLAYING AT LEAST ONE PHYSICAL, CHEMICAL OR BIOLOGICAL PROPERTY OF A TEST LIQUID
DISPOSITIF PERMETTANT DE DETERMINER ET D'AFFICHER AU MOINS UNE PROPRIETE PHYSIQUE, CHIMIQUE OU BIOLOGIQUE D'UN LIQUIDE TEST

(30) Priorität: 27.09.2002 DE 20214971 U
(43) Veröffentlichungstag der Anmeldung: 22.06.2005
(73) Patentinhaber: Klocke Verpackungs-Service GmbH, 76356 Weingarten (DE)
(72) Erfinder: KLOCKE, Hartmut, 76133 Karlsruhe (DE); LANGER, Heiko, 76356 Weingarten (DE)
(74) Vertreter: Frank, Gerhard
(86) Internationale Anmeldenummer: PCT/DE2003/003153
(87) Internationale Veröffentlichungsnummer: WO 2004/030821

(56) Entgegenhaltungen:
- EP-A- 0 803 288
- US-A- 3 620 676
- US-A- 3 768 978

## Beschreibung

### Technischer Hintergrund der Erfindung

Die Erfindung betrifft eine Vorrichtung zur Ermittlung und Anzeige mindestens einer physikalischen, chemischen oder biologischen Eigenschaft einer Testflüssigkeit oder zum Nachweis von in dieser enthaltenen Stoffe und/oder Lebewesen durch Reaktion mit mindestens einem Indikator oder Reaktionsmittel.

Die hier besonders interessierende Testflüssigkeit ist Wasser mit seinen physikalischen und chemischen Eigenschaften und seinen Inhaltsstoffen d. h. in der Regel im Wasser enthaltene Stoffe oder Stoffverbindungen, die dort als Zusatzstoffe beabsichtigt oder als Verunreinigungen unbeabsichtigt enthalten sind.

Wasser wird auf vielfältigste Weise genutzt, als Grundnahrungsmittel, in Industrie, aber auch zunehmend in unserer Freizeitgesellschaft. Wasser, als lebensnotwendiges Element, ist eine unserer wichtigste Ressourcen. Umweltfaktoren nehmen immer stärkeren Einfluss auf die Qualität unseres Wassers. Um dem Wasser oder anderen Flüssigkeiten bestimmte Eigenschaften zu verleihen, wie z. B. Schutz gegen Verkeimung oder Veränderung von Härtegraden ist es notwendig, dem Wasser bestimmte Zusatzstoffe beizumengen. Zu hohe Konzentrationen der Zusatzstoffe können schädlich, oder auf manche Organismen gar toxisch wirken.

Um größere Schäden an Mensch, Umwelt und Material zu verhindern, ist es notwendig einen ständigen Nachweis von bestimmten Inhaltsstoffen bzw. Mikroorganismen und deren Konzentration in Wasser und anderen Flüssigkeiten zu führen. Dabei sollten die Methoden zur Nachweisführung zuverlässig, einfach zu handhaben und reproduzierbar sein.

### Stand der Technik

Im Allgemeinen wird der Nachweis mittels eines Indikators geführt, der beim Zusammenbringen mit dem zu testenden Medium (Testflüssigkeit) zu bestimmten Reaktionen wie z. B. einem Farbumschlag führt. In vielen Fällen muss die zu testende Flüssigkeit zunächst aus einem größeren Behältnis entnommen und danach mit dem Indikator in einem bestimmten Volumenverhältnis zusammen geführt werden. Anhand einer separaten Vergleichstafel, wie z. b. Farbpalette werden dann die Inhaltsstoffe bzw. deren Konzentration festgestellt.

US 3,620,676 beschreibt eine Vorrichtung zur Probennahme einer Testflüssigkeit sowie zur Anzeige einer Eigenschaft dieser Flüssigkeit. Hierzu reagiert die aufgenommene Testflüssigkeit mit einem Indikator, der in der Reaktionskammer der Vorrichtung sitzt. Die Reaktionskammer ist dabei als Tiefziehnapf ausgebildet, der von einer Deckfolie verschlossen ist.

EP 0 803 288 A2 beschreibt eine Vorrichtung zur Probenahme und Analyse einer Flüssigkeit. Hierzu weist die Vorrichtung eine Kammer zur Erzeugung eines Unterdruckes auf, die über wenigstens einen Kanal und einer innerhalb desselben angeordneten Analysekammer mit einer Einsaugöffnung in Verbindung steht. Da Analysekammer dient dabei zur Aufnahmne eines Reagents und als Reaktionskammer des Reagents mit der zu untersuchenden Flüssigkeit.

### Beschreibung der Erfindung

Die Erfindung soll die Nachweisführung vereinfachen. Diese Aufgabe wird gemäß dem kennzeichnenden Teil des Schutzanspruchs 1 gelöst.

Der Grundgedanke der Erfindung ist darin zu sehen, dass die Testflüssigkeit ohne Zuhilfenahme eines Zwischenträgers entnommen werden kann und in der Reaktionskammer mit dem Indikator reagiert.

Die Reaktionskammer, in der der Nachweis durchgeführt wird, besteht gemäß einer besonders bevorzugten Ausgestaltung in ihrem Grundaufbau aus einer thermoplastisch zu einem Tiefziehnapf verformten Grundfolie mit aufgesiegelter Deckfolie und beinhaltet den zur Nachweisführung notwendigen Indikator, der vor dem Aktivieren gegen äussere Einflüsse wie z. B. Feuchtigkeit geschützt ist. Ein zur Nachweisführung erforderlicher Informationsträger, beispielsweise in Form eines Vergleichsstreifens, ist vorzugsweise direkt auf der Vorrichtung aufgebracht oder in eine separate Kammer eingelegt. Mit dieser einfachen Lösung kann die bekannte Technologie der Herstellung von Tiefziehverpackungen/Blisterpackungen für den gewünschten Zweck ausgestaltet und angewendet werden.

Weitere bevorzugte Ausgestaltungen sind weiteren Unteransprüchen zu entnehmen.

### Kurze Beschreibung der Zeichnungen

Mehrere Ausführungsbeispiele werden anhand von Zeichnungen näher erläutert, es zeigen:
Bild 1: Ansicht und Schnitt des ersten Ausführungsbeispiels,
Bild 2: ein erstes Ausführungsbeispiel in Perspektive,
Bild 3: die Handhabung des ersten Ausführungsbeispiels,
Bild 4: ein zweites Ausführungsbeispiel der erfindungsgemäßen Vorrichtung,
Bild 5: ein drittes Ausführungsbeispiel,
Bild 6: die Handhabung des dritten Ausführungsbeispiels,
Bild 7: ein vierten Ausführungsbeispiel mit 2 Kammern,
Bild 8: die Handhabung des vierten Ausführungsbeispiels,
Bild 9: ein fünftes Ausführungsbeispiel,
Bild 12: ein siebtes Ausführungsbeispiel,
Bild 13: Aufhängemöglichkeiten für die erfindungsgemäße Vorrichtung,
Bild 14: eine erste Aufstellmöglichkeit der erfindungsgemäßen Vorrichtung,
Bild 15: eine zweite Aufstellmöglichkeit der erfindungsgemäßen Vorrichtung,
Bild 16: eine dritte Aufstellmöglichkeit der erfindungsgemäßen Vorrichtung,
Bild 17: eine erste Variante der Ansaugöffnung ohne Sollbruchstelle, und
Bild 18: eine zweite Variante der Ansaugöffnung ohne Sollbruchstelle.

### Beschreibung der Ausführungsbeispiele

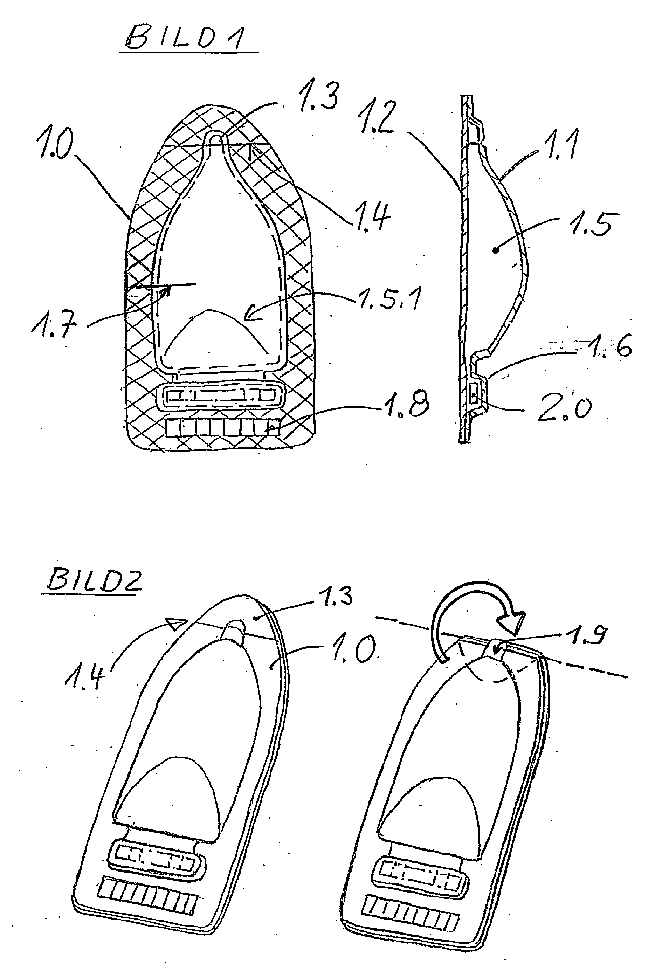

Bild 1 zeigt ein erstes Ausführungsbeispiel der erfindungsgemäßen Vorrichtung, die im folgenden zur Vereinfachung als Flüssigkeitstester bezeichnet ist. Der Flüssigkeitstester 1.0 besteht in seinem Grundaufbau aus einer zu einem Tiefziehnapf 1.1 thermoplastisch verformten Grundfolie und einer auf diesen aufgesiegelten Deckfolie 1.2 und bildet somit eine Tiefziehpackung. Der Tiefziehnapf 1.1 ist einseitig zu einer Abbrechnase 1.3, die zur späteren Entnahme der Testflüssigkeit dient, verjüngt und durch eine Sollbruchstelle (Rillung) 1.4 gegenüber der restlichen Tiefziehpackung abgetrennt. Zwischen Tiefziehnapf 1.1 und Deckfolie 1.2 ist somit eine Reaktionskammer 1.5 zur Aufnahme der Testflüssigkeit ausgebildet, und in einem Bereich 1.5.1 so gestaltet, dass sie nach einer Verformung durch Zusammenspiel von Formgebung und Materialwahl ihre ursprüngliche Form wieder einnimmt und somit ein Pumpmechanismus entsteht, in dem bei der Zurückbildung des verformten Tiefziehnapfs 1.1 sich ein Unterdruck aufbaut, der zu einer Ansaugwirkung auf die Eintrittsöffnung 1.9 nach abgebrochener Abbrechnase 1.3 führt.

Auf der gegenüberliegenden Seite der Abbrechnase 1.3 ist in einer separaten Indikatorkammer 1.6 ein streifenförmiger Indikator 2.0 eingelegt, der mit einem oder mehreren Reaktionsmitteln bestückt oder getränkt sein kann. Die Indikatorkammer 1.6 ist mit der Reaktionskammer 1.5 verbunden oder bildet einen Teil derselben, so dass die Testflüssigkeit in Kontakt mit dem Indikator 2.0 kommen kann.

Eine Markierung 1.7 ermöglicht es, das eingesogene Volumen der Testflüssigkeit zu erkennen. Sie kann sowohl als Tiefziehmarkierung oder Prägung in die Grundfolie eingebracht oder als Druck auf dem Tiefziehnapf 1.1 und/oder auf der Deckfolie 1.2 aufgebracht sein.

An geeigneter Stelle sind auf dem Flüssigkeitstester 1.0 ein oder mehrere Informationsträger als Vergleichsskalen in Form einer Farbpalette 1.8 aufgebracht. Die Farbpalette kann mittels eines Aufdrucks, aber auch durch Etikett realisiert sein.

Die beschriebenen Vergleichsskalen und die Volumenmarkierungen sind bei allen folgenden Ausführungsbeispielen anwendbar und werden daher dort nicht mehr erwähnt.

Bild 2 zeigt den Flüssigkeitstester 1.0 in Perspektive. Zur Aktivierung des Flüssigkeitstesters 1.0 wird er entlang seiner Sollbruchstelle 1.4 aufgebrochen, wodurch die Eintrittsöffnung 1.9 für die Testflüssigkeit freigelegt wird.

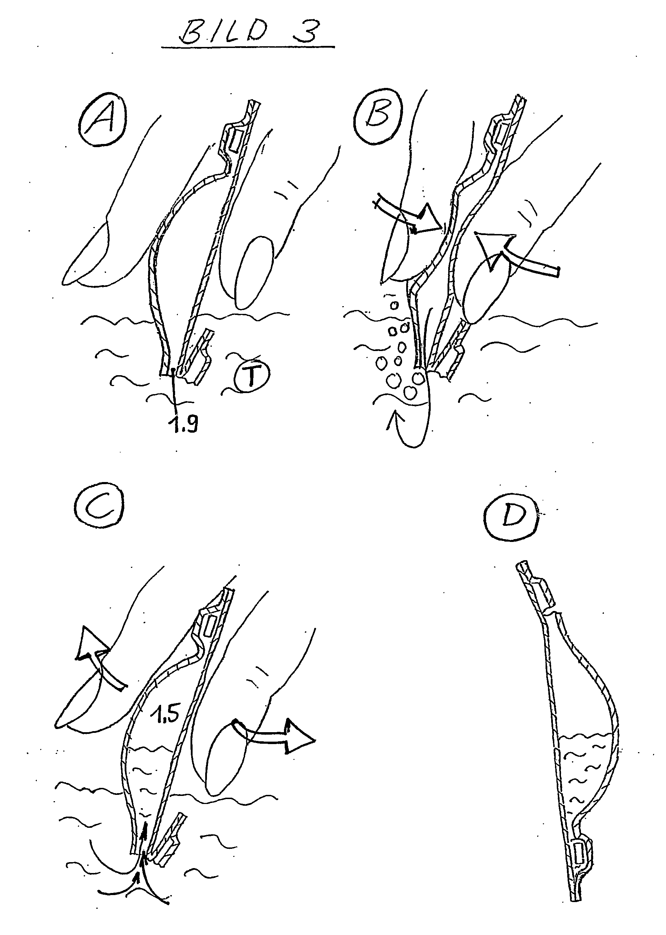

Bild 3 zeigt die verschiedenen Phasen der Entnahme der Testflüssigkeit T:
A: Die Eintrittsöffnung 1.9 des Flüssigkeitstesters 1.0 wird unter den Flüssigkeitsspiegel der Testflüssigkeit T gehalten.
B: Durch Druck auf die Reaktionskammer 1.5 entweicht die Luft aus dem Flüssigkeitstester 1.0.
C: Durch die Entlastung der Reaktionskammer 1.5 wird die Testflüssigkeit in die Reaktionskammer 1.5 eingesogen.
D: Nach dem Umdrehen des Flüssigkeitstesters 1.0 wird der Indikator 2.0 von der Testflüssigkeit umspült und reagiert mit dieser, wodurch sich beispielsweise eine Färbung einstellt, die mit den Farben auf der Vergleichsskala verglichen werden kann.

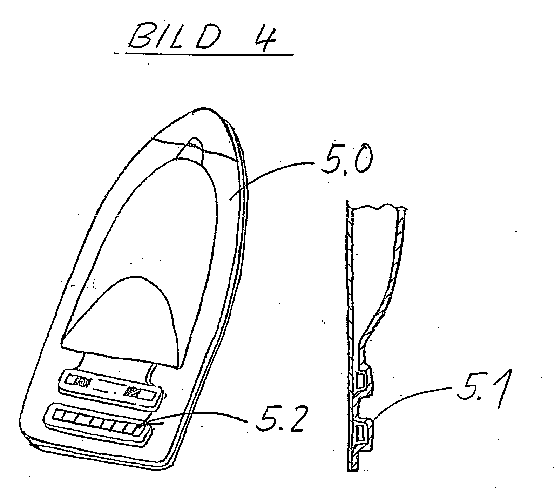

Bild 4 zeigt ein zweites Ausführungsbeispiel, den Flüssigkeitstester 5.0. Sein Grundaufbau ist identisch mit dem Flüssigkeitstester 1.0 und wird nicht näher beschrieben.

An geeigneter Stelle sind ein oder mehrere Tiefziehnäpfe 5.1 als Indikatorkammer angebracht, die gegenüber den anderen Kammern abgegrenzt sind und einen Indikator 5.2 in Form eines Vergleichsstreifens beliebiger Form aufnehmen können.

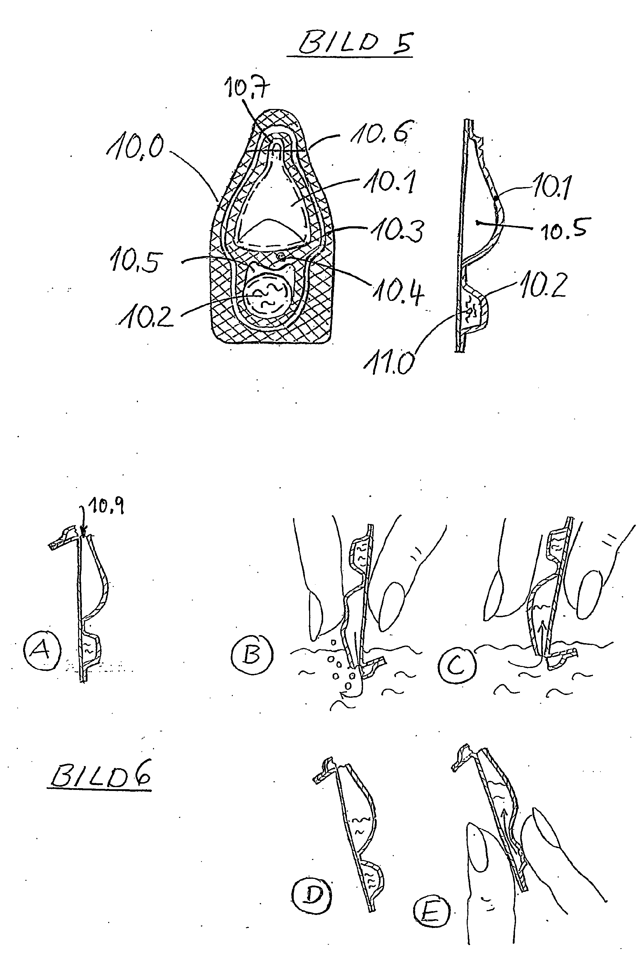

Bild 5 zeigt ein drittes Ausführungsbeispiel, den Flüssigkeitstester 10.0. Der Grundaufbau entspricht dem Flüssigkeitstester 1.0 und wird nicht näher beschrieben.

Der Flüssigkeitstester 10.0 trägt neben der Reaktionskammer 10.5 zum Einsaugen der Testflüssigkeit eine Indikatorkammer 10.2 zur Aufnahme eines Reaktionsmittels 11.0, das sowohl fest, flüssig oder in Pulverform sein kann. Die Kammern 10.1 und 10.2 sind von einer umlaufenden Festsiegelung 10.3 umgeben. Zwischen beiden Kammern erstreckt sich eine peelfähige Zone 10.4. Zur Erleichterung des Aufpeelens schließt sich an die Indikatorkammer 10.2 ein ungesiegelter Bereich 10.5 an.

Bild 6 zeigt die Anwendung des Flüssigkeitstesters 10.0.
A: Die Abbrechnase 10.7 des Flüssigkeitstesters wird entlang der Rillung 10.6 aufgebrochen.
B: Die Eintrittsöffnung 10.9 der geöffneten Abbrechnase 10.7 wird unter den Flüssigkeitsspiegel gehalten. Durch Druck auf die Reaktionskammer 10.1 entweicht Luft aus dem Flüssigkeitstester 10.0.
C: Durch die Entlastung der Reaktionskammer 10.1 wird Testflüssigkeit in die Reaktionskammer 10.1 des Flüssigkeitstesters 10.0 eingesogen.
D: Der Flüssigkeitstester 10.0 wird um ca. 180 Grad gedreht.
E: Durch Druck auf die Vorratskammer 10.2, die das Reaktionsmittel 11.0 beinhaltet, wird über die peelfähige Verbindung 10.4 eine Verbindung zwischen Reaktionskammer 10.1 und Vorratskammer 10.2 hergestellt. eine Reaktion von Testflüssigkeit und Reaktionsmittel 11.0 kann somit erfolgen.

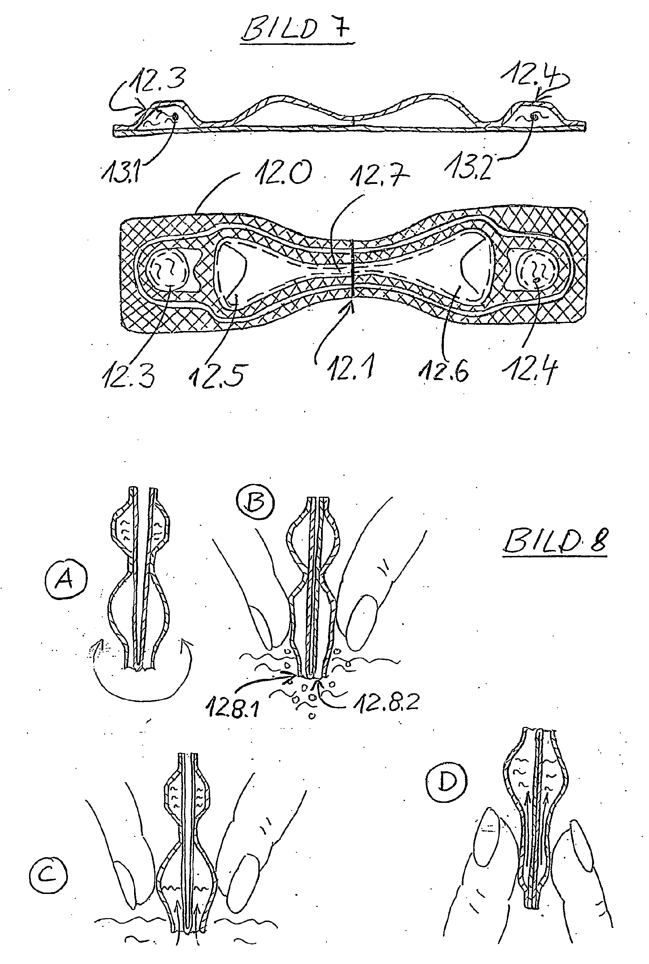

Bild 7 zeigt ein viertes Ausführungsbeispiel, den Flüssigkeitstester 12.0. Der Aufbau entspricht bis zu seiner Sollbruchstelle 12.1 dem Flüssigkeitstester 10.0 und wird nicht näher beschrieben.

Der Flüssigkeitstester 12.0 setzt sich ab der Sollbruchstelle 12.1 in spiegelbildlicher Form fort, so dass zwei Indikatorkammern 12.3 und 12.4 zur Aufnahme von, verschiedenen Reaktionsmitteln 13.1 und 13.2 gebildet werden. Die beiden Reaktionskammern 12.5 und 12.6, die zur Aufnahme der Testflüssigkeit dienen, sind durch einen gemeinsamen Abbrechkanal 12.7, der zum späteren Einsaugen der Testflüssigkeit dient, miteinander verbunden.

Bild 8 zeigt die Anwendung des Flüssigkeitstesters 12.0.
A: Der Flüssigkeitstester 12.0 wird entlang seiner Sollbruchstelle 12.1 aufgebrochen; die flachen Seiten des Flüssigkeitstesters 12.0 zueinander geklappt.
B: Die entstehenden Eintrittsöffnungen 12.8.1 und 12.8.2 werden unter den Flüssigkeitsspiegel gehalten. Durch gleichzeitigen Druck auf die Reaktionskammern 12.5 und 12.6 entweicht die Luft aus den Reaktionskammern.
C: Durch die Entlastung der Reaktionskammern 12.5 und 12.6 wird die Testflüssigkeit in den Flüssigkeitstester 12.0 eingesogen.
D: Nach dem Drehen des Flüssigkeitstesters um ca. 180 Grad wird durch gleichzeitigen Druck auf die Vorratskammern 12.3 und 12.4 die peelfähige Verbindung zwischen den Kammern 12.3 und 12.5 bzw. zwischen den Kammern 12.4 und 12.6 gelöst, so dass die Reaktionsmittel 13.1 und 13.2 mit der Testflüssigkeit reagieren können.

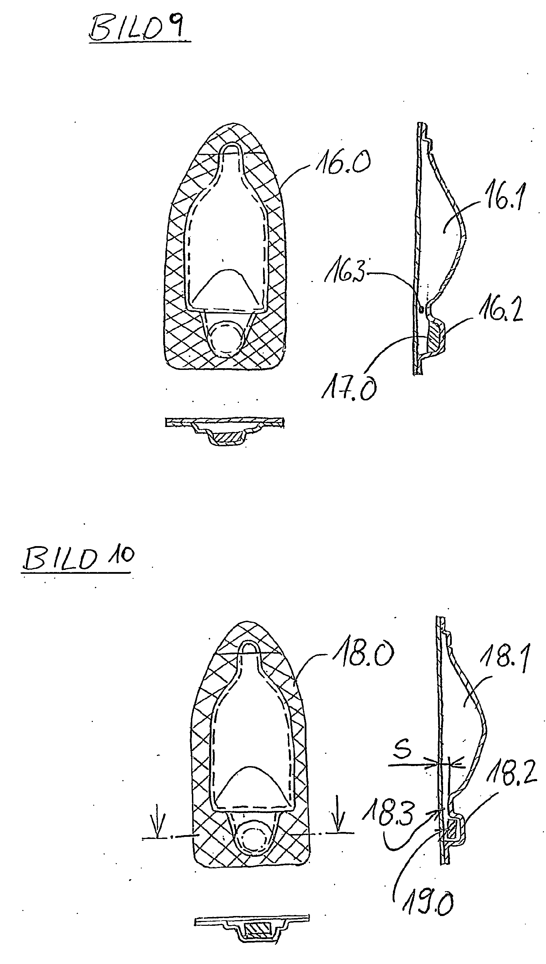

Bild 9 zeigt ein fünftes Ausführungsbeispiel, den Flüssigkeitstester 16.0. Der Grundaufbau entspricht dem Flüssigkeitstester 1.0 und wird nicht näher beschrieben.

Die Reaktionskammer 16.1 schließt sich eine Indikatorkammer 16.2 an, die geeignet ist, ein pastöses oder beim Einbringen flüssiges, danach aber erstarrendes Reaktionsmittel 17.0 aufzunehmen. Die Kammern 16.1 und 16.2 sind im Bereich 16.4 zueinander offen, so dass bei der Anwendung das Reaktionsmittel 17.0 mit der zu testenden Flüssigkeit reagieren kann.

Bild 10 zeigt ein sechstes Ausführungsbeispiel, den Flüssigkeitstester 18.0. Der Aufbau entspricht dem Flüssigkeitstester 16.0.

Der Reaktionskammer 18.1, die zur Aufnahme der Testflüssigkeit dient, schließt sich eine Indikatorkammer 18.2 an, die ein Reaktionsmittel 19.0 in fester Form aufnimmt. Beide Kammern sind im Bereich 18.3 zueinander offen. Die Indikatorkammer 18.2 ist so gestaltet, dass bei der Anwendung das Reaktionsmittel von der Testflüssigkeit umspült werden kann. Der Spalt S der Öffnung 18.3 ist so gewählt, dass das Reaktionsmittel 19.0 in der Indikatorkammer 18.2 festgehalten wird.

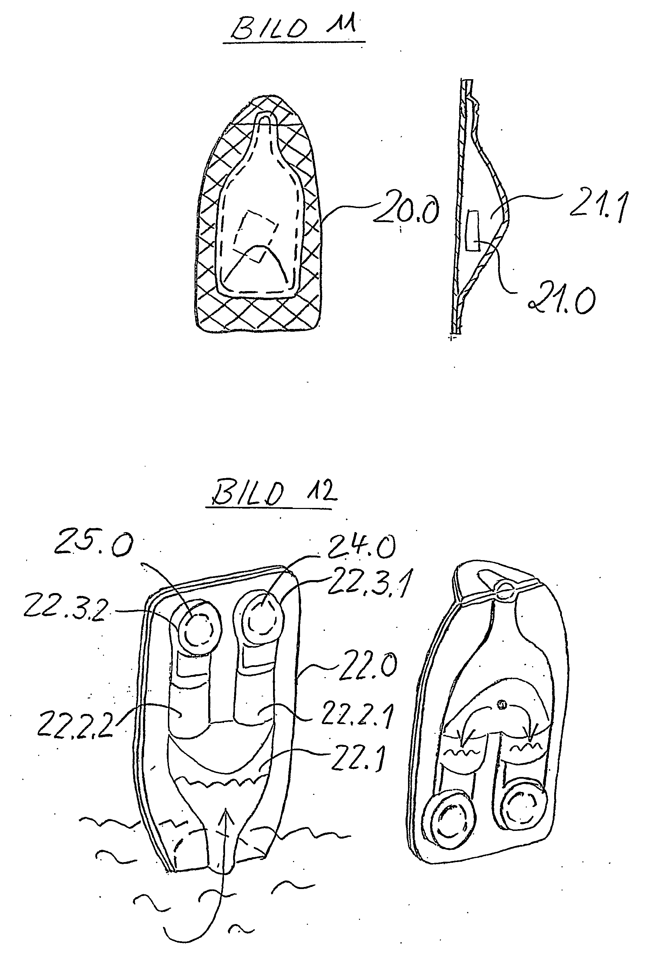

Bild 12 zeigt ein siebtes Ausführungsbeispiel, den Flüssigkeitstester 22.0. Der Grundaufbau entspricht dem Flüssigkeitstester 1.0 und wird nicht näher beschrieben.

Die Reaktionskammer 22.1, die zur Aufnahme der Testflüssigkeit dient, setzt sich in zwei Röhren 22.2.1 und 22.2.2 fort, welche an ihrem Ende zu Indikatorkammern 22.3.1 und 22.3.2 ausgebildet sind, die Indikatoren 24.0 und 25.0 in Form von Trägern mit einem Reaktionsmittel aufnehmen. Mit dem Flüssigkeitstester 22.0 können somit gleichzeitig verschiedene Messwerte der Testflüssigkeit ermittelt und aufgezeigt werden.

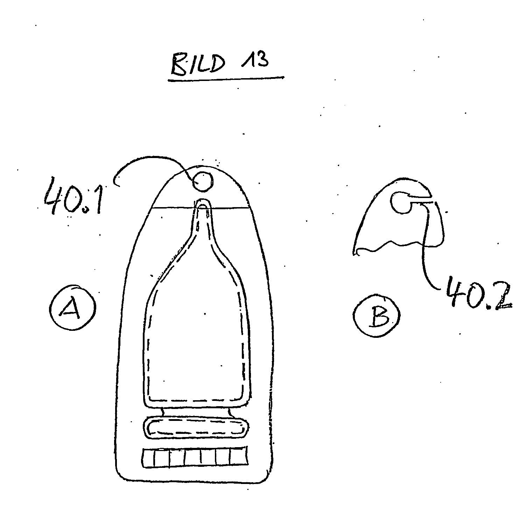

Bild 13 zeigt Aufhängemöglichkeiten des Flüssigkeitstesters gemäß einem der oben beschriebenen Ausführungsbeispiele:
A: Durch eine rundum geschlossene Stanzung 40.1 im Endbereich des Flüssigkeitstesters.
B: Durch eine Stanzung 40.2 im Endbereich des Flüssigkeitstesters, die zur Außenkante hin offen ist.

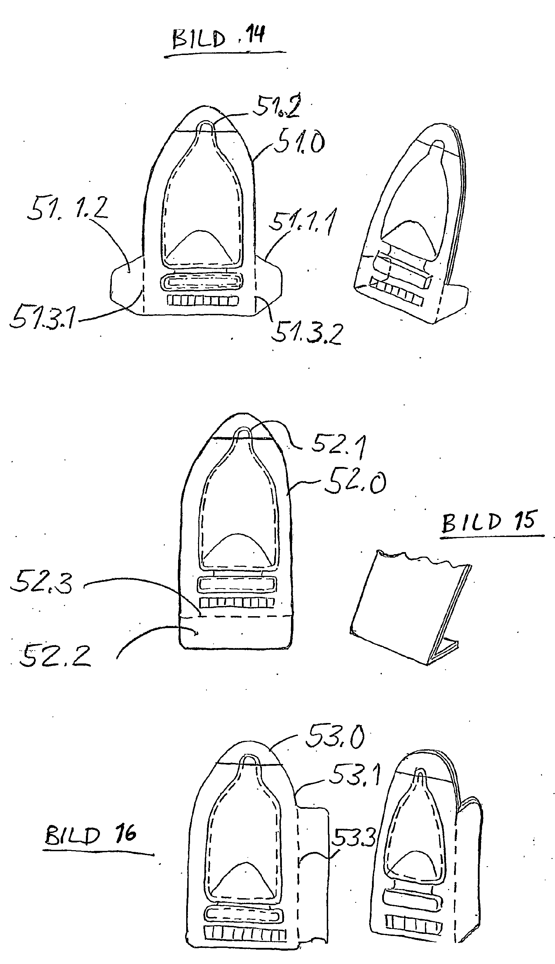

Bild 14 zeigt eine erste Möglichkeit zur Aufstellung des Flüssigkeitstesters 51.0:

Der Flüssigkeitstester 51.0 ist durch seitliche Laschen 51.1.1 und 51.1.2 auf der der Abbrechnase 51.2 abgewandten Seite verbreitert und durch Perforationen 51.3.1 und 51.3.2 von diesen abgegrenzt. Durch Umknicken der Laschen 51.1.1 und 51.1.2 entlang der Perforationslinien 51.3.1 und 51.3.2 kann der Flüssigkeitstester in vertikaler Position stabil aufgestellt werden.

Bild 15 zeigt eine zweite Möglichkeit zur Aufstellung des Flüssigkeitsspenders 52.0:

Der Flüssigkeitstester 52.0 ist auf der der Abbrechnase 52.1 abgewandten Seite durch eine Lasche 52.2 verlängert und durch eine Perforation 52.3, die rechtwinklig zur Längsachse des Flüssigkeitstesters 52.0 verläuft, abgegrenzt. Durch das Umklappen der Lasche 52.2 entlang der Perforationslinie 52.3 ergibt sich eine Standmöglichkeit für den Flüssigkeitstester.

Bild 16 zeigt eine dritte Möglichkeit zur Aufstellung des Flüssigkeitstesters 53.0:

Der Flüssigkeitstester 53.0 ist entlang seiner Seitenkante 53.1 einseitig durch eine seitliche Lasche 53.2 verbreitert und durch eine Perforation 53.3 gegenüber dem Flüssigkeitstester abgegrenzt. Durch das Umklappen der Lasche 53.2 entlang der Perforationslinie 53.3 ergibt sich eine stabile Aufstellmöglichkeit für den Flüssigkeitstester 53.0.

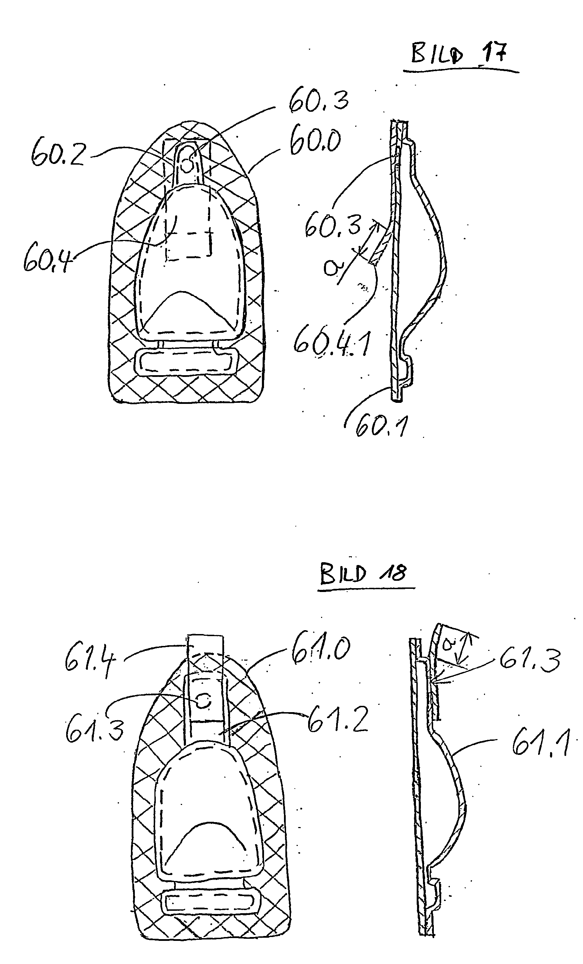

Bild 17 zeigt eine erste Variante einer Ansaugöffnung ohne Sollbruchstelle als vierzehntes Ausführungsbeispiel des Flüssigkeitstesters.

Die Deckfolie 60.1 des Flüssigkeitstesters 60.0 hat im Bereich seiner Ansaugnase 60.2 eine Eintrittsöffnung 60.3, die mit einer Klebelasche 60.4 abgedeckt ist. Die Klebelasche 60.4 ist im Bereich -a- kleberfrei und zu einer Abziehlasche 60.4.1 ausgebildet. Vor der Nutzung des Flüssigkeitstesters 60.0 wird die Klebelasche 60.4 abgezogen.

Bild 18 zeigt eine zweite Variante der Ansaugöffnung ohne Sollbruchstelle als fünfzehntes Ausführungsbeispiel.

Die Bodenfolie 61.1 des Flüssigkeitstesters 61.0 ist im Bereich der Ansaugnase 61.2 mit einer Stanzung 61.3 versehen. die Ansaugnase 61.2 ist so gestaltet, dass sie ein Klebeetikett 61.4 aufnehmen kann. Das Klebeetikett 61.4 deckt die Eintrittsöffnung 61.3 ab und ist in seinem überstehenden Bereich -a- kleberfrei. Vor der Nutzung des Wassertesters 61.0 wird das Klebeetikett 61.4 abgezogen.

## Patentansprüche

1. Vorrichtung zur Ermittlung und Anzeige mindestens einer physikalischen, chemischen oder biologischen Eigenschaft einer Testflüssigkeit oder zum Nachweis von in dieser enthaltenen Stoffe und/oder Lebewesen durch Reaktion mit mindestens einem Indikator oder Reaktionsmittel, wobei der/die Indikator(en) (2.0) zumindest teilweise in oder über mindestens eine(r) Reaktionskammer (1.5) zugänglich ist/sind, die zumindest eine Eintrittsöffnung für die, Testftüssigkeit aufweist/aufweisen und als mindestens ein Tiefziehnapf (1.1) ausgebildet ist, der mit einer Deckfolie (1.2) verschlossen ist, **dadurch gekennzeichnet, dass** mindestens ein Indikator (2.0) auf der der Eintrittsöffnung (1.9) gegenüberliegenden Seite der Reaktionskammer (1.5) in einer separaten Indikatorhammer angeordnet ist.

2. Vorrichtung nach Anspruch 1, **dadurch gekennzeichnet, dass** Form und/oder Materialeigenschaften der Reaktionskammer(n) derart gewählt sind, dass nach einer Verformung durch einen äußeren Druck zur Reduzierung des ursprünglichen Volumens der Reaktionskammer bei Wegnahme der Verformungsbeaufschlagung ein Rückstelleffekt derart erzeugt wird, dass die ursprüngliche Form unter Erzeugung einer Einsaugwirkung wieder soweit hergestellt wird, dass das ursprüngliche Volumen zumindest im wesentlichen wiederhergestellt wird.

3. Vorrichtung nach Anspruch 2, **dadurch gekennzeichnet, dass** der Rückstelleffekt durch Wahl der Form und der Eigenschaften des Kunststoffmaterials des Tiefziehnapfes (1.1) erreicht ist.

4. Vorrichtung nach Anspruch 1, **dadurch gekennzeichnet, dass** der Indikator (2.0) ein Teststreifen ist.

5. Vorrichtung nach Anspruch 1, **dadurch gekennzeichnet, dass** der Indikator (11.0) mindestens eine Flüssigkeit ist und die die Indikatorkammer (10.2) mit der Reaktionskammer (10.5) verbindbar ist.

6. Vorrichtung nach Anspruch 1, **dadurch gekennzeichnet, dass** der Indikator (15.0) der mindestens ein Feststoff ist, der in der Indikatorkammer (14.2) bereitgehalten ist, zu der die Testflüssigkeit Zugang hat, oder von der aus er in die Reaktionskammer (14.1) gelangen kann.

7. Vorrichtung nach Anspruch 1, **dadurch gekennzeichnet, dass** der Randbereich, in dem Tiefziehnapf (1.1) und Deckfolie (1.2) miteinander durch Siegelung verbunden sind, Ausformungen und/oder Einformungen (40.1, 40.2) aufweist, die ein Aufstellen oder Aufhängen gestatten.

8. Vorrichtung nach Anspruch 1, **dadurch gekennzeichnet, dass** in unmittelbarer Nachbarschaft des Indikators oder der Reaktionskammer mindestens ein Informationsträger (1.8) vorgesehen ist, der die möglichen Zustände des Indikators, nach der Reaktion mit der Testflüssigkeit wiedergibt.

9. Vorrichtung nach Anspruch 4 und 8, **dadurch gekennzeichnet, dass** der Informationsträger ein Vergleichsteststreifen ist, der in einem weiteren Tiefziehnapt (1.6) angeordnet ist, der an die Reaktionskammer (1.5) angrenzt.

10. Vorrichtung nach Anspruch 1, **dadurch gekennzeichnet, dass** die Eintrittsöffnung (1.9) durch eine Abbrechnase (1.3) gebildet ist, die mit der Reaktionskammer (1.5) verbunden ist, und über die eine quer verlaufende Materialschwächung z.B. in Form einer Rillung (1.4) verläuft.

11. Vorrichtung nach Anspruch 1, **dadurch gekennzeichnet, dass** die Eintrittsöffnung durch eine Stanzung (60.3, 61.3) gebildet ist, die mit einer Klebefolie (60.4, 61.2) abgedeckt ist.

12. Vorrichtung nach Anspruch 1 und 10, **dadurch gekennzeichnet, dass** zwei Reaktionskammem (12.5, 12.6) und zwei Indikatorkammern (12.3; 12.4) vorgesehen sind, die derart gegenüberliegend angeordnet sind, dass die beiden Reaktionskammern über einen gemeinsamen Kanal (12.7) verbunden sind, über den die Materialschwächung zur Bildung von zwei benachbarten Eintrittsöffnungen (12.8.1; 12.8.2) für die beiden Reaktionskammern verläuft.

13. Vorrichtung nach Anspruch 5 oder 6, **dadurch gekennzeichnet, dass** Reaktionskammer (10.5) und Indikatorkammer (10.2) über eine peelfähige Zone (10.4) von Tiefziehnapf und Deckfolie miteinander verbindbar sind.

## Claims

1. Device for the determining and displaying of at least one physical, chemical or biological property of a test liquid or for the detection of substances and/or living organisms through reaction with at least one indicator or reactant, whereby the indicator(s) (2.0) is/are at the least partly accessible in or via at least one reaction chamber (1.5), which has/have at least one inlet port (1.9) for the test liquid and is/are in the form of at least one deep-drawn bowl (1.1), which is sealed with a protective foil (1.2), **characterized in that** at least one indicator (2.0) is positioned on the side of the reaction chamber (1.5) that is opposite to the inlet port (1.9) in a separate indicator chamber.

2. Device as per Claim 1, **characterized in that** the shape and/or material properties of the reaction chamber(s) are selected so that after a forming process due to an external pressure for the reduction of the original volume of the reaction chamber has taken place, when the forming impact is removed a resetting effect is produced so that the original shape is once again reproduced when being subjected to an inward suction to the extent that the original volume is at least essentially reproduced.

3. Device as per Claim 2, **characterized in that** the resetting effect is achieved through selecting the shape and the properties of the plastic material of the deep-drawn bowl (1.1).

4. Device as per Claim 1, **characterized in that** the indicator (2.0) is a test strip.

5. Device as per Claim 1, **characterized in that** the indicator (11.0) is a liquid and the indicator chamber (10.2) can be connected to the reaction chamber (10.5).

6. Device as per Claim 1, **characterized in that** the indicator (15.0) is at least a solid material, which is held in readiness in the indicator chamber (14.2), to which the test liquid(s) has access or from out of which it can get into the reaction chamber (14.1).

7. Device as per Claim 1, **characterized in that** the border area, in which the deep-drawn bowl (1.1) and the protective foil (1.2) are connected by sealing, has drawn and/or and moulded parts (40.1 and 40.2), which permit a mounting or suspension.

8. Device as per Claim 1, **characterized in that** at least one information carrier (1.8) is fitted in the direct vicinity of the indicator or of the reaction chamber, which reflects the possible conditions of the indicator in accordance with the reaction with the test liquid.

9. Device as per Claims 4 and 8, **characterized in that** the information carrier is a comparison test strip, which is fitted in a further deep drawn bowl (1.6), which is adjacent to the reaction chamber (1.5).

10. Device as per Claim 1, **characterized in that** the inlet port (1.9) is formed from a breakaway lug (1.3), which is connected to the reaction chamber (1.5) and runs for example in the form of a groove (1.4) over the transverse running material weakening.

11. Device as per Claim 1, **characterized in that** the inlet port is formed by a pressing (60.3, 61.3), which is covered with an adhesive foil (60.4, 61.2).

12. Device as per Claims 1 and 10, **characterized in that** both reaction chambers (12.5 and 12.6) are and two indicator chambers (12.3, 12.4) are fitted, which are positioned opposite each other and **in that** the two reaction chambers are connected by a common conduit (12.7), over which the material weakening runs for the development of two neighbouring inlet ports (12.8.1, 12.8.2) for the two reaction chambers.

13. Device as per Claim 5 or 6, **characterized in that** reaction chamber (10.5) and indicator chamber (10.2) can be connected to each other via a peelable area (10.4) of deep drawn bowl and protective foil.

## Revendications

1. Dispositif pour déterminer et afficher au moins une propriété physique, chimique ou biologique d'un liquide test, ou pour identifier des substances et/ou des organismes contenus dans celui-ci, par réaction avec au moins un indicateur ou un réactif, le/les indicateur(s) (2.0) étant au moins partiellement accessibles dans ou par l'intermédiaire d'au moins une chambre de réaction (1.5) qui présente/présentent au moins une ouverture d'entrée pour le liquide test et qui est réalisée par au moins un godet embouti (1.1) fermé par un film de recouvrement (1.2), **caractérisé en ce qu'**au moins un indicateur (2.0) est agencé du côté de la chambre de réaction (1.5) opposé à l'ouverture d'entrée (1.9), dans une chambre à indicateur séparée.

2. Dispositif selon la revendication 1, **caractérisé en ce que** la forme et/ou les propriétés du matériau de la/des chambre(s) de réaction sont choisies de façon que suite à une déformation par une pression externe pour réduire le volume initial de la chambre de réaction, il se produise à l'enlèvement de la sollicitation de déformation, un effet de rappel tel que la forme initiale est reconstituée avec formation d'un effet d'aspiration, à tel point que le volume initial est, au moins pour l'essentiel, reconstitué.

3. Dispositif selon la revendication 2, **caractérisé en ce que** l'effet de rappel est obtenu par le choix de la forme et des propriétés du matériau synthétique du godet embouti (1.1).

4. Dispositif selon la revendication 1, **caractérisé en ce que** l'indicateur (2.0) est une bande de test.

5. Dispositif selon la revendication 1, **caractérisé en ce que** l'indicateur (11.0) est au moins un liquide et la chambre à indicateur (10.2) peut être reliée avec la chambre de réaction (10.5).

6. Dispositif selon la revendication 1, **caractérisé en ce que** l'indicateur (15.0) est au moins une matière solide qui est tenue à disposition dans la chambre à indicateur (14.2) à laquelle le liquide test a accès, ou depuis laquelle l'indicateur peut atteindre la chambre de réaction (14.1).

7. Dispositif selon la revendication 1, **caractérisé en ce que** la zone en bordure, dans laquelle le godet embouti (1.1) et le film de recouvrement (1.2) sont reliés ensemble par scellement, présente des empreintes ou évidements (40.1, 40.2) qui permettent la position dressée ou l'accrochage.

8. Dispositif selon la revendication 1, **caractérisé en ce qu'**au voisinage immédiat de l'indicateur ou de la chambre de réaction, il est prévu au moins un support d'information (1.8) qui restitue les états possibles de l'indicateur après la réaction avec le liquide test.

9. Dispositif selon la revendication 4 et 8, **caractérisé en ce que** le support d'information est une bande de test comparative qui est agencée dans un autre godet embouti (1.6) adjacent à la chambre de réaction (1.5).

10. Dispositif selon la revendication 1, **caractérisé en ce que** l'ouverture d'entrée (1.9) est formée par un bec de rupture (1.3) qui est relié à la chambre de réaction (1.5) et en travers duquel s'étend un affaiblissement de matière, par exemple sous la forme d'une rainure (1.4).

11. Dispositif selon la revendication 1, **caractérisé en ce que** l'ouverture d'entrée est formée par un poinçonnage (60.3, 61.3) qui est recouvert par un film adhésif (60.4, 61.2).

12. Dispositif selon la revendication 1 et 10, **caractérisé en ce qu'**il est prévu deux chambres de réaction (12.5, 12.6) et deux chambres à indicateur (12.3 ; 12.4) agencées l'une en face de l'autre de façon que les deux chambres de réaction soient reliées par l'intermédiaire d'un canal (12.7) commun, sur lequel passe l'affaiblissement de matière pour former deux ouvertures d'entrée (12.8.1 ; 12.8.2) voisines pour les deux chambres de réaction.

13. Dispositif selon la revendication 5 ou 6, **caractérisé en ce que** chambre de réaction (10.5) et chambre à indicateur (10.2) peuvent être reliées ensemble par l'intermédiaire d'une zone (10.4) pelable de godet embouti et de film de recouvrement.
